# FASCICULE DE BREVET EUROPEEN

(11) **EP 1 792 567 B1**
(45) Date de publication et mention de la délivrance du brevet: **10.08.2011**
(21) Numéro de dépôt: 06291861.0
(22) Date de dépôt: 01.12.2006
(51) Int. Cl.: A61B 5/103

(54) **Appareil de detection du syndrome du canal carpien**
Gerät zur Erkennung eines Karpaltunnelsyndroms
Device for detecting carpal tunnel syndrome

(30) Priorité: 02.12.2005 FR 0512231
(43) Date de publication de la demande: 06.06.2007
(73) Titulaire: Université de Technologie de Belfort-Montbelliard (UTBM), 90400 Sevenans (FR)
(72) Inventeur: Sagot, Jean-Claude, 70400 Chagey (FR); Gomes, Samuel, 25490 Badevel (FR); Touvenot, Gérard, 70290 Plancher Bas (FR)
(74) Mandataire: Nithardt, Roland

(56) Documents cités:
- EP-A- 0 364 158
- WO-A-00/59377
- US-A- 4 250 891
- US-A- 5 027 828
- JENG ONE-JANG ET AL: "Gap detection tactility test for sensory deficits associated with carpal tunnel syndrome" ERGONOMICS; ERGONOMICS DEC 1995 TAYLOR & FRANCIS LTD, BASINGSTOKE, ENGL, vol. 38, no. 12, décembre 1995 (1995-12), pages 2588-2601, XP008068873

## Description

### Domaine technique :

La présente invention concerne un appareil permettant de détecter les signes précoces du Syndrome du Canal Carpien (SCC), pathologie faisant partie des Troubles Musculo-Squelettiques (TMS), première maladie professionnelle (MP) déclarée (presque 70% des MP), qui relève du tableau n°57 de la CNAMTS (Caisse Nationale d'Assurance Maladie des Travailleurs salariés), cet appareil comportant un boîtier pourvu d'au moins deux ouvertures, chacune communiquant avec une zone de réception agencée pour recevoir en appui la dernière phalange d'un doigt d'un sujet et des moyens mécaniques de mesure de la sensibilité cutanée dans chaque zone de réception.

### Technique antérieure :

Les entreprises notamment du secteur de l'industrie manufacturière sont particulièrement confrontées à des problèmes de santé au travail et en particulier à des problèmes d'hyper sollicitation de l'appareil locomoteur qualifiés de Troubles Musculo-Squelettiques (TMS) et reconnus comme maladie professionnelle. Les causes des TMS sont d'origines multiples: répétitivité des gestes, angulations extrêmes des segments des membres supérieurs, efforts importants, organisation du travail, stress, etc., et nécessitent souvent une intervention chirurgicale une fois la pathologie déclarée. La pathologie la plus fréquemment déclarée est le Syndrome du Canal Carpien (SCC) détecté le plus souvent par le salarié lui-même ressentant au niveau de la main des douleurs nocturnes : fourmillements, perte de sensibilité et plus particulièrement au niveau de la pulpe des doigts (extrémité de la dernière phalange) et/ou douleurs dans la paume de la main qui peuvent irradier vers le poignet. Le médecin du travail pratique un examen clinique qui consiste en un interrogatoire et en des tests neurologiques. Si le symptôme est avéré, le patient est dirigé vers un neurologue spécialiste Utilisant des techniques de stimulodétection à l'aide d'un électromyogramme (EMG) Cet examen permet d'enregistrer l'activité ou la conductivité électrique spontanée d'un muscle ou d'un nerf et se pratique en cabinet médical ou en milieu hospitalier, Il se déroule sur une durée allant de 20 à 40 minutes et nécessite la pose d'électrodes, soit invasives sous la forme d'aiguilles insérées sous la peau sur le trajet d'un nerf et à son contact, soit non invasives sous la forme d'électrodes appliquées sur la peau entre deux points distants sur un trajet nerveux. Cet examen confirme l'examen clinique et est généralement suivi d'une intervention chirurgicale. Si une affection du poignet liée au syndrome du canal carpien est Lion traitée, elle peut évoluer vers le coude (Epicondylites et Epitrochleites) puis vers l'épaule (Scapulalgies et Penarthrites) par hyper sollicitation de ces articulation. A l'inverse, l'affection liée au syndrome du canal carpien disparaît si, dès l'apparition des premiers signes de la maladie, le sujet n'est plus soumis aux sollicitations ayant conduit à l'apparition de cette pathologie.

Il existe donc un besoin en termes de détection précoce du syndrome du canal carpien dans le but de repérer notamment les postes de travail et les activités professionnelles à problème afin de déclencher les plans d'action adéquate pour supprimer les causes et réduire, voire supprimer la maladie, notamment en agissant sur l'organisation du travail et/ou en aménageant les postes de travail, etc. L'enjeu est important du point de vue des économies substantielles susceptibles d'être effectuées, en coûts directs et indirects, par les entreprises, les caisses d'assurance maladie, etc.

Il n'existe à ce jour aucun appareil de détection, rapide et peu onéreux, permettant d'aboutir à une évaluation précise et précoce du syndrome du canal carpien.

On connaît par ailleurs, certains outils manuels utilisés par les praticiens en cabinet pour tester la sensibilité cutanée à différents endroits du corps et détecter la diminution de la conductible nerveuse dans le cas de certaines maladies comme le diabète, la sclérose en plaque, etc. Ces outils comportent des protubérances soit en forme de dents dont l'intervalle est variable, soit en forme de tiges dont le diamètre est différent, ces protubérances étant appliquées sur les zones de corps pour tester la sensibilité cutanée par le pouvoir discriminant. Ces outils tels que décrits dans les publications EP 364.158, US 5,027,828 et US 6,387,055 restent artisanaux dans leur utilisation et l'interprétation des résultats par le praticien est sujet à caution. Ils ne donnent pas de résultats mesurables et ne permettent pas une reproductibilité des tests, ni un suivi épidémiologique.

On connaît également certains outils automatiques ou semi-autocnatiques comme ceux décrits dans les publications WO 00/59377 et US 4,250,891 qui ne comportent qu'un seul test de sensibilité, soit par pression cutanée, soit par discrimination ne permettant pas d'obtenir des résultats précis ni d'estimer le degré de la pathologie. Notamment, la publication WO 00/59377 propose un appareil de détection de la perte de sensibilité cutanée pourvu d'un boîtier muni d'une ouverture pour accueillir l'extrémité d'un doigt, dont la pulpe est sollicitée par un dispositif de mesure de la sensibilité par pression cutanée, et d'un bouton d'acquisition de la réponse du patient, cet appareil pouvant être connecté à un ordinateur pour l'analyse des résultats.

### Exposé de l'invention :

La présente invention propose une solution à ce problème en fournissant un appareil capable de détecter les signes précoces du syndrome du canal carpien, de manière rapide, par exemple en moins de 5 minutes, au moyen d'une technique simple, économique et non invasive, cet appareil étant portatif et pouvant être utilisé dans différents milieux même non hospitalier, voire par des non-praticiens, et permettant de faire un dépistage régulier afin d'identifier d'une part les organisations et les postes de travail à risques afin de les faire évoluer, voire les re-concevoir, et d'autre part les personnes susceptibles d'être atteintes pour les diriger vers un neurologue. L'objectif recherché est de pouvoir différencier les différents stades de la maladie : des stades précoces aux stades reconnus pathologiques, et de doter le médecin, l'infirmièr(e), de façon générale, les professions de santé concernées par des questions de conditions de vie au travail, d'un appareil fonctionnel, performant et fiable leur permettant ainsi d'aider les entreprises à mieux évaluer les conséquences de leur choix de conception des postes de travail, d'organisation du travail, de faire face au vieillissement des salariés en permettant le maintien dans l'emploi, de faciliter la féminisation de l'emploi, de conduire une politique de santé active en réduisant de manière significative cette pathologie.

Dans ce but, l'invention concerte un appareil de détection du syndrome du canal carpien comportant au moins un dispositif de mesure de la sensibilité par pression cutanée associé à une première zone de réception et un dispositif de mesure de la sensibilité par discrimination cutanée associé à une seconde zone de réception, ledit dispositif de mesure de la sensibilité par pression cutanée comportant un élément déformable monté pivotant à l'une de ses extrémités, l'autre extrémité portant un organe d'appui destiné à être en contact avec le doigt du sujet, et une came rotative autour d'un axe excentré et couplée à un mécanisme d'entraînement en rotation, cette came étant disposée au contact d'un bras de transmission solidaire de l'élément déformable pour modifier la pression d'appui dudit organe d'appui, ledit dispositif de mesure de la sensibilité par discrimination cutanée comportant un organe rotatif, couplé audit mécanisme d'entraînement en rotation, et comportant en périphérie des discontinuités de surface, les intervalles entre deux discontinuités de surface adjacentes étant différents les uns des autres, lesdits mécanismes d'entraînement comportant un actionneur commun, et ledit appareil comportant des moyens de traitement des mesures fournies par lesdilts dispositifs de mesure de la sensibilité cutanée agencés pour donner un résultat représentatif d'un niveau de sensibilité.

Ainsi, la position du doigt du sujet est toujours au même endroit par rapport aux moyens mécaniques de contrôle, ce qui permet de supprimer le côté aléatoire des outils manuels utilisés par, les praticiens, et la combinaison d'au moins deux tests différents de mesure de là sensibilité cutanée par pression et par discrimination permet d'évaluer avec précision la sensibilité de la pulpe d'un doigt implique dans le syndrome du canal carpien et d'estimer le degré de la pathologie impossible à atteindre avec les outils automatiques existants, cette méthode étant très simple, rapide et peu coûteuse.

Ce dispositif comporte avantageusement des moyens de détection de la pression d'appui dudit organe d'appui, par exemple sous la forme d'une jauge de contrainte fixée sur l'élément déformable.

Ces discontinuités de surface peuvent être en relief et formées par des protubérances plus ou moins espacées ou de dimensions plus ou moins grandes. Elles peuvent être aussi en creux et formées par les arêtes bordant des cavités plus ou moins espacées ou de dimensions plus ou moins grandes.

L'organe rotatif est de préférence une roue dont la surface périphérique comporte des encoches plus ou moins larges formant les discontinuités de surface. Ce dispositif comporte avantageusement des moyens de détection desdites discontinuités, par exemple sous la forme d'un capteur de positionnement fixé sur l'organe rotatif.

De préférence, la came rotative et l'organe rotatif sont avantageusement portés par un arbre de réception commun couplé audit actionneur commun.

De manière avantageuse, l'appareil comporte des moyens de réponse agencés pour être commandés par le sujet en fonction de ce qu'il perçoit, par exemple au moyen d'un bouton d'acquisition commun aux dits dispositifs de mesure de la sensibilité

### Description sommaire des dessins :

La présente invention et ses avantages apparaîtront mieux dans la description suivante d'un mode de réalisation donné à titre d'exemple non limitatif, en référence aux dessins annexés, dans lesquels :
   - la figure 1 est une vue en perspective de l'appareil selon l'invention,
   - la figure 2 est une vue schématique du principe de fonctionnement du dispositif de mesure de la sensibilité par pression cutanée,
   - la figure 3 est une vue schématique du principe de fonctionnement du dispositif de mesure de la sensibilité par discrimination cutanée,
   - la figure 4 est une vue en perspective de l'intérieur de l'appareil de la figure 1,
   - la figure 5 est une vue de détail en perspective des deux dispositifs de mesure visibles sur la figure 4, et
   - la figure 6 est un exemple de diagramme de résultats d'un sujet suivi sur une période donnée.

### Illustrations de l'invention :

Le syndrome du canal carpien (SCC) se caractérise par une perte de la sensibilité au niveau de la main, et plus particulièrement dans la zone d'extrémité des doigts où la densité de récepteurs nerveux est importante, notamment au niveau de l'index ou du majeur. Il est ainsi possible d'établir une estimation du degré de la pathologie en examinant uniquement la sensibilité de la pulpe des doigts, localisée au niveau de la dernière phalange de l'index ou du majeur.

Le principe de fonctionnement de l'appareil de détection 1 du syndrome du canal carpien selon l'invention s'appuie sur la combinaison de deux tests simples de sensibilité : un test de pression cutanée 2 pour mesurer la sensibilité à la pression mécanique sur un doigt, et un test de discrimination cutanée 3 pour mesurer le pouvoir discriminant de ce même doigt, afin d'obtenir des résultats complémentaires permettant d'évaluer avec une plus grande précision la sensibilité du doigt testé et d'estimer le degré de la pathologie.

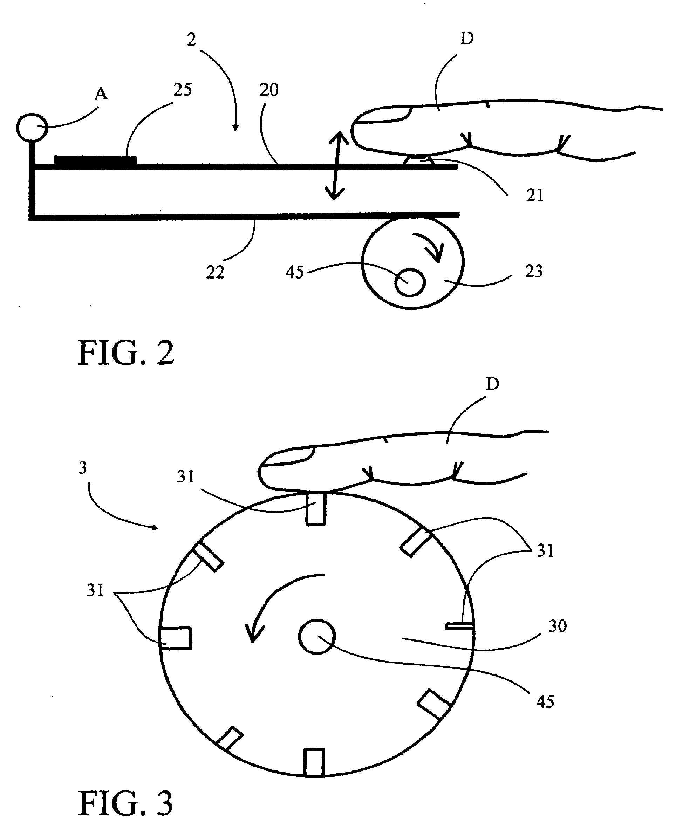

Le test de pression cutanée 2 est représenté schématiquement à la figure 2. Il se caractérise par une mesure de déformation d'un élément déformable 20, par exemple métallique, allongé, à section rectangulaire, sous une charge que l'on fait varier jusqu'à ce que la personne testée, appelée par la suite le sujet, ressente le contact d'un organe d'appui 21 tel qu'une pastille, une protubérance, une pointe ou similaire, sur son doigt D. La donnée mesurée est l'effort exercé sur le doigt D.

Le test de la discrimination cutanée 3 est représenté schématiquement à la figure 3. Il est basé sur la détermination du pouvoir discriminant du sujet quant à l'importance d'une discontinuité de surface. On fait tourner un organe rotatif 30 comportant plusieurs discontinuités de surface 31, comme des encoches, de tailles différentes et on identifie celles que le sujet est capable de ressentir sur son doigt D. La donnée mesurée est la largeur de la plus petite encoche 31 détectée par le sujet.

### Meilleure manière de réaliser l'invention :

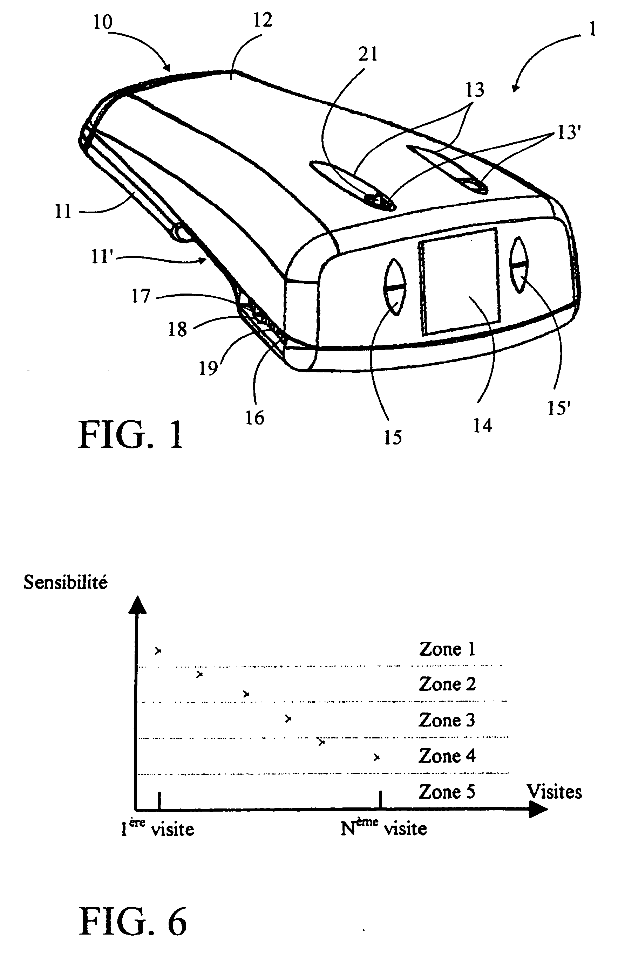

L'appareil de détection 1 du syndrome du canal carpien est décrit en référence aux figures 1 à 5. Il comporte, en référence à la figure 1, un boîtier 10 renfermant les moyens mécaniques de contrôle de la sensibilité cutanée, à savoir un dispositif de mesure de la sensibilité par pression cutanée 2 selon la figure 2 et un dispositif de mesure de la sensibilité par discrimination cutanée 3 selon la figure 3. Ce boîtier 10 est réalisé de préférence en matière synthétique moulée telle que du PVC et formé de deux coques 11, 12 assemblées, par exemple par vissage, rivetage, clippage ou similaire. Ces deux coques définissent une platine 11 pour porter tous les composants et un capot 12 pour couvrir la platine 11. Ce capot 12 comporte en face supérieure deux empreintes allongées, sensiblement parallèles, formant des zones de réception 13 agencées pour recevoir en appui plan, la pulpe d'un doigt D d'un sujet et plus précisément la partie inférieure de la dernière phalange de l'index ou du majeur. Lorsqu'elle est en appui sur ces zones de réception 13, la pulpe du doigt D est soumise aux dispositifs de mesure 2, 3 au travers d'ouvertures 13' ménagées dans ces zones de réception 13. Le boîtier 10 comporte, en face avant, un écran de lecture 14 des menus proposés et des résultats des tests et deux boutons de commande 15, 15' des menus. Par exemple, le bouton 15 du haut sert à monter dans le menu relatif au choix du test retenu et à la transmission des données vers un ordinateur, le bouton 15 du bas sert à descendre dans le menu ; le bouton 15' du haut sert à valider le choix du menu et le bouton 15' du bas sert à revenir au menu précédent, ces fonctions n'étant pas limitatives. Ce boîtier 10 comporte sur ses deux côtés deux poignées 11' de transport. Il comporte également, sur un de ses côtés, un bouton marche/arrêt 16, une prise 17 à laquelle est raccordée par un câble un bouton d'acquisition (non représenté) pour recueillir la réponse du sujet lors des tests, une prise électrique 18 pour alimenter l'appareil de détection 1 par un câble électrique branché sur le secteur (non représenté), et une prise d'ordinateur 19 pour le raccorder à un micro-ordinateur permettant le transfert et la gestion des résultats.

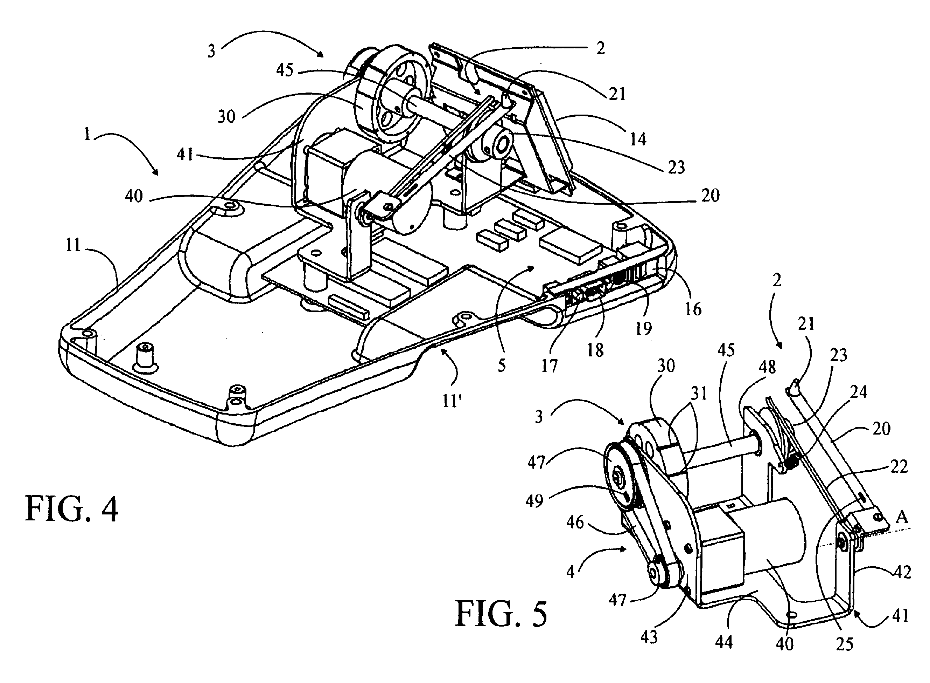

L'intérieur du boîtier 10 est représenté à la figure 4. Il comporte un sous-ensemble constituant les moyens mécaniques de contrôle de la sensibilité cutanée illustré plus en détail à la figure 5. Ce sous-ensemble comporte un mécanisme d'entraînement 4 pourvu d'un actionneur 40 tel qu'un moteur électrique, un moto-réducteur, un moteur pas à pas, un servomoteur, ou similaire, monté sur un support 41 en forme de U, définissant un fond 44 destiné à être fixé sur la platine 11, par exemple par vissage, rivetage, clippage ou similaire, et deux parois latérales 42, 43. Ce mécanisme d'entraînement 4 entraîne un arbre récepteur 45 en rotation par l'intermédiaire d'une transmission à courroie 46 et poulies 47, cet arbre récepteur 45 étant guidé en rotation par deux paliers 48 prévus dans les parois latérales 42, 43 du support 41. La transmission peut être constituée d'autres moyens mécaniques tels que pignons et chaîne, poulies et courroie crantée, train d'engrenage, bielle et manivelle, pignons et crémaillère, etc. permettant de réduire la vitesse de l'arbre récepteur 45 par rapport à celle de l'actionneur 40. Il est aussi possible de prévoir un actionneur 40 en prise directe avec l'arbre récepteur 45 ou tout autre mécanisme d'entraînement en rotation adéquat.

Le dispositif de mesure de la sensibilité par pression cutanée 2 comporte un élément déformable 20 doublé d'un bras de transmission 22 sensiblement parallèle, le tout monté pivotant autour d'un axe de rotation A solidaire du support 41, l'extrémité libre de l'élément déformable 20 comportant un organe d'appui 21 sous la forme d'une protubérance conique ou de toute autre forme adéquate. L'élément déformable 20 est par exemple métallique et l'organe d'appui 21 est en matière synthétique moulée telle que du PVC. Ce dispositif 2 comporte également une came rotative 23 excentrée et couplée à l'arbre récepteur 45. L'extrémité libre de ce bras de transmission 22 repose sur le sommet de cette came rotative 23 qui, lorsqu'elle tourne, le déplace en translation alternative de haut en bas se transformant en un mouvement de pivotement alternatif de l'élément allongé déformable 20 autour de son axe de rotation A. Ce bras de transmission 22 est sollicité en direction de la came rotative 23 par un organe de rappel 24 tel qu'un ressort de traction ou tout autre organe adapté, disposé entre le bras de transmission 22 et la paroi latérale 42 correspondante. Lorsque la came rotative 23 parcourt un tour complet, elle soulève l'élément déformable 20 sur le premier demi-tour et l'abaisse sur le second demi-tour. Lorsque l'élément déformable 20 se soulève, l'organe d'appui 21 exerce une pression croissante sur la pulpe du doigt D du sujet placé dans la zone de réception 13 correspondante. Dès que le sujet perçoit une pression, il appuie sur le bouton d'acquisition avec sa main libre. L'appareil de détection 1 enregistre l'intensité de la pression de l'organe d'appui 21 perceptible par le sujet. Cette pression est mesurée par des moyens de détection formés par exemple d'une jauge de contrainte 25 fixée directement sur l'élément déformable 20 pour mesurer sa déformation ou tout autre capteur adapté mécanique, optique, électromagnétique, par rayonnements, etc.

Le dispositif de mesure de la sensibilité par discrimination cutanée 3 comporte un organe rotatif 30, tel qu'une roue par exemple métallique, monté directement sur l'arbre récepteur 45 et pourvu, en périphérie, de discontinuités de surface 31 discriminantes, les intervalles entre deux discontinuités 31 adjacentes étant différents les uns des autres. Ces discontinuités 31 sont, dans l'exemple illustré, constituées par les arêtes bordant des encoches de largeurs différentes ménagées dans la surface périphérique de l'organe rotatif 30. Ces discontinuités 31 peuvent également être formées par des reliefs de protubérances ou par des creux de cavités, plus ou moins espacés et/ou de dimensions plus ou moins importantes. Lorsque l'organe rotatif 30 tourne, les encoches 31 discriminantes effleurent la pulpe du doigt D du sujet placée dans la zone de réception 13 correspondante. A chaque fois que le sujet perçoit une de ces encoches 31, il appuie sur le bouton d'acquisition avec sa main libre. L'appareil de détection 1 enregistre l'encoche 31 la plus fine perceptible par le sujet qui est identifiée par des moyens de détection formés par exemple d'un capteur de positionnement 49 fixé sur l'organe rotatif 30 qui peut être résistif, magnétique, capacitif, optique, ou autre. Ces moyens de détection peuvent aussi être constitués par l'actionneur 40 lui-même s'il s'agit d'un moteur pas à pas.

L'appareil de détection 1 comporte une carte électronique 5 incorporant un logiciel et agencée à la fois pour piloter l'actionneur 30 et traiter les résultats des tests, à savoir les signaux émis par les moyens de détection des deux dispositifs de mesure 2 et 3 en fonction des signaux reçus du bouton d'acquisition, indiquer un niveau de sensibilité en correspondance avec les stades de la maladie en fonction d'une échelle préenregistrée, mémoriser les mesures de tests par sujet, etc. L'actionneur 30 et la carte électronique 5 sont alimentés par le secteur au moyen d'un câble électrique raccordé à la prise électrique 18. L'appareil de détection 1 peut également être équipé d'une batterie (non représentée) lui permettant d'être autonome en cas de besoin. La carte électronique 5 est également raccordable à un micro-ordinateur ou similaire, par la prise d'ordinateur 19 qui peut être un port série RS232, un port USB, un port IEEE, etc. de manière à pouvoir récupérer les résultats des tests, les stocker, effectuer un traitement statistique des résultats, etc. Ce micro-ordinateur peut comporter une base de données spécifique permettant de rassembler toutes les informations relatives aux sujets testés, afin d'assurer le suivi de l'évolution des symptômes pour chaque sujet, chaque service, chaque entreprise, etc.

### Possibilités d'application industrielle :

Le fonctionnement de l'appareil de détection 1 selon l'invention est à présent décrit. Après la mise sous tension de l'appareil de détection 1 au moyen du bouton marche/arrêt 16, le médecin,l'infirmier(e) ou autre personne relevant des professions de la santé, lance le début des tests à l'aide des boutons de commande 15, 15' et de l'écran de contrôle 14 situés à l'avant du boîtier 10. Le sujet effectue alors, selon un protocole précis, les deux tests destinés à évaluer sa sensibilité : le test de pression cutanée 2 ainsi que le test de discrimination cutanée 3. Pour ce faire, il pose à tour de rôle la dernière phalange de son index ou de son majeur sur les zones de réception 17 au sommet du boîtier 10. Dans un premier temps, il peut effectuer avec sa main droite, le test de pression cutanée 2 suivi du test de discrimination cutanée 3 situé juste à côté, puis dans un second temps, il change de main pour effectuer à nouveau ces mêmes tests. Au cours de ces deux tests, le sujet dispose dans sa main libre d'un bouton d'acquisition. Ainsi, dès qu'il perçoit sous son doigt D le contact de l'organe d'appui 21 pour le premier test 2 ou une encoche 31 pour le second test 3, il le signale en appuyant sur le bouton d'acquisition. Le traitement des résultats s'effectue à la fin des tests et le logiciel incorporé à la carte électronique 5 de l'appareil de détection 1 détermine le niveau de sensibilité du sujet et le cas échéant l'évolution de ce niveau de sensibilité par rapport à la précédente visite. L'appareil de détection 1 peut être couplé à un micro-ordinateur permettant d'avoir une véritable gestion des résultats de tests. Un exemple des résultats obtenus est illustré à la figure 6 sous la forme d'un diagramme représentant par des points, le niveau de sensibilité d'un sujet à chaque visite. L'échelle des niveaux de sensibilité est découpée en plusieurs zones, par exemple de 1 à 5 allant d'une sensibilité dite normale à une perte de sensibilité et correspondant aux différents stades de la maladie. Le logiciel permettant l'interprétation des résultats des tests comporte une échelle élaborée à partir de campagnes de mesures menées sur un panel de sujets représentatifs et étalonnées au moyen d'un électromyogramme.

Ainsi, il ressort clairement de cette description que l'invention permet d'atteindre les buts fixés, à savoir un appareil de détection 1 simple, facile d'utilisation, permettant à l'aide de tests complémentaires non invasifs, de suivre l'évolution de la sensibilité cutanée des personnes laissant supposer qu'elles pourraient être atteintes à terme des symptômes du syndrome du canal carpien si elles continuent à être exposées aux mêmes hyper sollicitations des membres supérieurs au cours de leur activité de travail, à la même organisation, etc. Cet appareil de détection 1 s'inscrit parfaitement dans une démarche de prévention visant à conduire une politique de santé active autour des TMS, à réduire de manière conséquente les coûts directs et indirects liés à ce type de pathologie, et à faire face ainsi à un vieillissement des salariés en permettant le maintien dans l'emploi.

L'invention est décrite en référence essentiellement au syndrome du canal carpien dans le cadre d'une démarche de prévention des maladies professionnelles mais s'étend à toute autre application dans laquelle il est utile de détecter de manière précoce une carence de la sensibilité nerveuse.

La présente invention n'est pas limitée à l'exemple de réalisation décrit mais s'étend à toute modification et variante évidentes pour un homme du métier tout en restant dans l'étendue de la protection définie dans les revendications annexées.

## Revendications

1. Appareil, de détection (1) du syndrome du canal carpien, comportant un boîtier (10) pourvu d'au moins demi ouvertures (13') chacune communiquant avec une zone de réception (13) agencée pour recevoir en appui la dernière phalange d'un doigt (D) impliqué dans le syndrome du canal carpien d'un sujet, et, d'au moins, un dispositif de mesure de la sensibilité par pression cutanée (2) associé à une première zone de réception (13) et un dispositif de mesure de la sensibilité par discrimination cutanée (3) associé à une seconde zone de réception (13), ledit dispositif de mesure de la sensibilité par pression cutanée (2) comportant un élément déformable (20) monté pivotant à l'une de ses extrémités, l'autre extrémité portant un organe d'appui (21) destiné à être en contact avec le doigt (D) du sujet, et une came rotative (23) autour d'un axe excentre et couplée à un mécanisme d'entraînement (4) en rotation, cette came rotative (23) étant disposée au contact d'un bras de transmission (22) solidaire dudit élément déformable (20) pour modifier la pression d'appui dudit organe d'appui (21), ledit dispositif de mesure de la sensibilité par discrimination cutanée (3) comportant un organe rotatif (30), couplé à un mécanisme d'entraînement (4) en rotation, ledit organe rotatif comportant en périphérie des discontinuités de surface (31) les intervalles entre deux discontinuités de surface (31) adjacentes étant différents les uns des autres lesdits mécanismes d'entraînement (4) en rotation des deux dispositifs de mesure de sensibilité comportant un actionneur (40) commun, et ledit appareil (1) comportant des moyens de traitement des mesures fournies par lesdits dispositifs de mesure de la sensibilité cutanée (2, 3) agencés pour donner un résultat représentatif d'un niveau de sensibilité.

2. Appareil selon la revendication 1, **caractérisé en ce que** le dispositif de mesure de la sensibilité par pression cutanée (2) comporte des moyens de détection de la pression d'appui dudit organe d'appui (21).

3. Appareil selon la revendication 2, **caractérisé en ce que** lesdits moyens de détection de la pression d'appui comportent une jauge de contrainte (25) fixée sur ledit élément déformable (20).

4. Appareil selon la revendication 1, **caractérisé en ce que** ledit organe d'appui (21) est une protubérance sensiblement conique.

5. Appareil selon la revendication 1, **caractérisé en ce que** les discontinuités de surface (31) sont en relief et formées de protubérances plus ou moins espacées et/ou de dimensions plus ou moins grandes.

6. Appareil selon la revendication 1, **caractérise en ce que** les discontinuités de surface (31) sont en creux et formées par les arêtes bordant des cavités plus ou moins espacées et/ou de dimensions plus ou moins importantes.

7. Appareil selon la revendication 6, **caractérisé en ce que** l'organe rotatif (30) est une roue dont la surface périphérique comporte des encoches plus ou moins larges formant lesdites discontinuités de surface (31).

8. Appareil selon la revendication 1, **caractérise en ce que** le dispositif de mesure de la sensibilité par discrimination cutanée (3) comporte des moyens de détection desdites discontinuités de surface (31).

9. Appareil selon la revendication 8, **caractérisé en ce que** les moyens de détection comportent un capteur de positionnement (49) fixé sur ledit organe rotatif (30).

10. Appareil selon la revendication 1, **caractérisé en ce que** la came rotative (23) et organe rotatif (30) sont portés par un arbre de réception (45) commun couplé audit actionneur (40) commun.

11. Appareil selon la revendication 1, **caractérise en ce qu'**il comporte des moyens de réponse agences pour être commandés par le sujet en fonction de ce qu'il perçoit.

12. Appareil selon la revendication 11, **caractérisé en ce que** les moyens de réponse comportent un bouton d'acquisition commun aux dits dispositifs de mesure de la sensibilité (2, 3).

## Claims

1. Device (1) for detecting the carpal tunnel syndrome, comprising a housing (10) having at least two openings (13') communicating each with one receiving area (13) arranged for placing on it the last phalanx (D) of a finger involved in the carpal tunnel syndrome of a subject, and at least one sensitivity measuring device by skin pressure (2) associated with a first receiving area (13), and a sensitivity measuring device by tactile discrimination (3) associated with a second receiving area (13), said sensitivity measuring device by skin pressure (2) comprising a deformable element (20) mounted so as to pivot at one of its ends, the other end carrying a pressing element (21) intended for coming in contact with the finger (D) of the subject, and a cam (23) rotating around an eccentric axis coupled with a rotational driving mechanism (4), this rotary cam (23) being arranged in contact with a transmission arm (22) firmly attached to said deformable element (20) in order to modify the pressure exerted by said pressing element (21), said sensitivity measuring device by tactile discrimination (3) comprising a rotary element (30) coupled with a rotational driving mechanism (4), said rotary element comprising on its periphery surface discontinuities (31), all intervals between two adjacent surface discontinuities (31) being different from each other, said rotational driving mechanisms (4) of the two sensitivity measuring devices comprising a common drive (40), and said device (1) comprising processing means for the measurements supplied by said skin sensitivity measuring devices (2, 3), arranged so as to give a result representative of a sensitivity level.

2. Device according to claim 1, **characterised in that** the sensitivity measuring device by skin pressure (2) comprises means for detecting the pressure exerted by said pressing element (21).

3. Device according to claim 2, **characterised in that** said means for detecting the pressure exerted comprise a strain gauge (25) fastened on said deformable element (20).

4. Device according to claim 1, **characterised in that** said pressing element (21) is an approximately conical protuberance.

5. Device according to claim 1, **characterised in that** the surface discontinuities (31) are in relief and formed by protuberances more or less spaced and/or with more or less large dimensions.

6. Device according to claim 1, **characterised in that** the surface discontinuities (31) are recessed and formed by the edges limiting cavities more or less spaced and/or with more or less large dimensions.

7. Device according to claim 6, **characterised in that** the rotary element (30) is a wheel whose peripheral surface comprises more or less wide notches forming said surface discontinuities (31).

8. Device according to claim 1, **characterised in that** the sensitivity measuring device by tactile discrimination (3) comprises means for detecting said surface discontinuities (31).

9. Device according to claim 8, **characterised in that** the means for detecting comprise a positioning sensor (49) fastened on said rotary element (30).

10. Device according to claim 1, **characterised in that** the rotary cam (23) and the rotary element (30) are carried by a common shaft (45) coupled with said common drive (40).

11. Device according to claim 1, **characterised in that** it comprises answering means arranged so as to be controlled by the subject according to what he perceives.

12. Device according to claim 11, **characterised in that** the answering means comprise an acquisition button common to said sensitivity measuring devices (2, 3).

## Patentansprüche

1. Gerät (1) zur Erkennung eines Karpaltunnelsyndroms, bestehend aus einem Gehäuse (10) mit mindestens zwei Öffnungen (13'), wobei jede dieser mit einem Aufnahmebereich (13) in Verbindung steht, der ausgelegt ist, um das letzte Fingerglied (D) eines von dem Karpaltunnelsyndroms betroffenen Fingers einer Person darauf zu legen, und aus mindestens einer Vorrichtung zum Messen der Empfindlichkeit durch Ausübung eines Drucks auf die Haut (2), verbunden mit einem ersten Aufnahmebereich (13), und einer Vorrichtung zur Messung der Empfindlichkeit über die taktile Unterscheidungsfähigkeit (3), verbunden mit einem zweiten Aufnahmebereich (13), wobei die besagte Vorrichtung zum Messen der Empfindlichkeit durch Ausübung eines Drucks auf die Haut (2) ein verformbares Element (20) beträgt, das an einem seiner Enden drehbar gelagert ist, und dessen anderes Ende ein mit dem Finger (D) der Person in Berührung kommendes Druck-Organ (21), sowie einen um eine exzentrisch angeordnete Achse drehenden und mit einem Drehantriebsmechanismus (4) gekoppelten Drehnocken (23) aufweist, wobei dieser Drehnocken (23) mit einem fest mit dem besagten verformbaren Element (20) verbundenen Übertragungsarm (22) in Berührung ist um die Druckkraft des besagten Druck-Organs (21) zu ändern, wobei die besagte Vorrichtung zur Messung der Empfindlichkeit über die taktile Unterscheidungsfähigkeit (3) ein mit einem Drehantriebsmechanismus (4) gekoppeltes Drehelement (30) beträgt, wobei das besagte Drehelement auf seinem Umfang Oberflächen-Unterbrechungen (31) aufweist, wobei alle Intervalle zwischen zwei anliegenden Oberflächen-Unterbrechungen (31) unterschiedlich sind, wobei die besagten Drehantriebsmechanismen (4) der beiden Empfindlichkeits-Messvorrichtungen einen gemeinsamen Antrieb (40) aufweisen, und das besagte Gerät (1) Mittel zur Verarbeitung der von den besagten Vorrichtungen zur Messung der taktilen Empfindlichkeit (2, 3) gelieferten Messungen betragen, die ausgelegt sind, um ein für ein Empfindlichkeits-Niveau repräsentatives Ergebnis zu geben.

2. Gerät nach Anspruch 1, **dadurch gekennzeichnet, dass** die Vorrichtung zum Messen der Empfindlichkeit durch Ausübung eines Drucks auf die Haut (2) Mittel zur Erfassung des von dem besagten Druck-Organ (21) ausgeübten Drucks beträgt.

3. Gerät nach Anspruch 2, **dadurch gekennzeichnet, dass** die besagten Mittel zur Erfassung des Drucks einen an dem besagten verformbaren Element (20) befestigten Dehnungsmessstreifen (25) betragen.

4. Gerät nach Anspruch 1, **dadurch gekennzeichnet**, das besagte Druck-Organ (21) eine merklich konische Protuberanz ist.

5. Gerät nach Anspruch 1, **dadurch gekennzeichnet, dass** die Oberflächen-Unterbrechungen (31) erhaben sind und von mehr oder weniger entfernten und/oder mehr oder weniger großen Protuberanzen gebildet sind.

6. Gerät nach Anspruch 1, **dadurch gekennzeichnet, dass** die Oberflächen-Unterbrechungen (31) vertieft sind und von den Kanten von mehr oder weniger entfernten und/oder mehr oder weniger großen Vertiefungen gebildet sind.

7. Gerät nach Anspruch 6, **dadurch gekennzeichnet, dass** das Drehelement (30) ein Rad ist, dessen Umfangsfläche mehr oder weniger breite Kerben beträgt, die die besagten Oberflächen-Unterbrechungen (31) bilden.

8. Gerät nach Anspruch 1, **dadurch gekennzeichnet, dass** die Vorrichtung zur Messung der Empfindlichkeit über die taktile Unterscheidungsfähigkeit (3) Mittel zur Erfassung der besagten Oberflächen-Unterbrechungen (31) beträgt.

9. Gerät nach Anspruch 8, **dadurch gekennzeichnet, dass** die Mittel zur Erfassung einen am besagten Drehelement (30) befestigten Positionierungs-Sensor (49) betragen.

10. Gerät nach Anspruch 1, **dadurch gekennzeichnet, dass** der Drehnocken (23) und das Drehelement (30) von einem gemeinsamen Schaft (45) getragen werden, der mit dem besagten gemeinsamen Antrieb (40) gekoppelt ist.

11. Gerät nach Anspruch 1, **dadurch gekennzeichnet, dass** es Antwort-Mittel beträgt, die ausgelegt sind, um von der Person je nach seinen Empfindungen betätigt zu werden.

12. Gerät nach Anspruch 11, **dadurch gekennzeichnet, dass** die Antwort-Mittel einen den beiden besagten Mitteln zur Messung der Empfindlichkeit (2, 3) gemeinsamen Erfassungs-Knopf betragen.
